# FASCICULE DE BREVET EUROPEEN

(11) **EP 3 946 523 B1**
(45) Date de publication et mention de la délivrance du brevet: **26.02.2025**
(21) Numéro de dépôt: 20723918.7
(22) Date de dépôt: 03.04.2020
(51) Int. Cl.: A61M 5/32, A61M 5/50, A61M 5/31

(54) **DISPOSITIF D'INJECTION DE PRODUIT FLUIDE**
FLUIDPRODUKTINJEKTIONSVORRICHTUNG
FLUID PRODUCT INJECTION DEVICE

(30) Priorité: 04.04.2019 FR 1903626
(43) Date de publication de la demande: 09.02.2022
(73) Titulaire: APTAR France SAS, 27110 Le Neubourg (FR)
(72) Inventeur: PETIT, Ludovic, 27110 Vitot (FR)
(74) Mandataire: CAPRI
(86) Numéro de dépôt international: PCT/FR2020/000088
(87) Numéro de publication internationale: WO 2020/201645

(56) Documents cités:
- US-A- 5 151 088
- US-A- 6 110 147
- US-A1- 2004 171 991
- US-B1- 6 428 519

## Description

La présente invention concerne un dispositif d'injection de produit fluide, et plus particulièrement un tel dispositif pourvu d'un dispositif de sécurité.

Les dispositifs de sécurité pour seringue d'injection, notamment les seringues du type pré-remplies, sont bien connus. Le document FR2922112 décrit un exemple d'un tel dispositif de l'état de la technique.

Ces dispositifs de sécurité comportent généralement un manchon externe assemblé autour de la seringue, qui est adapté à se déployer en fin d'injection pour recouvrir l'aiguille. Ces dispositifs nécessitent généralement de nombreuses pièces mobiles et/ou déformables, tels que des ressorts et des pattes flexibles, ce qui rend la fabrication et l'assemblage relativement complexes. L'utilisation d'un ou plusieurs ressort(s) et de pièces plastiques déformables peut générer des risques de dysfonctionnement, notamment après un long temps de stockage, pendant lequel le ressort peut perdre de sa force dynamique et les pattes flexibles peuvent perdre leur flexibilité ou élasticité.

Les documents US2004171991, US6428519, US6110147 et US5151088 décrivent d'autres dispositifs de l'état de la technique. Tous ces dispositifs prévoient un manchon mobile disposé à l'intérieur de la seringue et adapté à être poussé manuellement en fin de course d'actionnement vers une position de sécurité dans laquelle il recouvre l'aiguille. Ces dispositifs sont relativement complexes et donc coûteux à fabriquer et à assembler.

La présente invention a pour but de fournir un dispositif d'injection qui ne reproduit pas les inconvénients susmentionnés.

La présente invention a aussi pour but de fournir un dispositif d'injection qui soit fiable d'utilisation, qui soit sûr et qui empêche tout risque de blessure, et qui soit simple et peu coûteux à fabriquer et à assembler.

La présente invention a donc pour objet un dispositif d'injection de produit fluide comportant :
- une seringue comportant un réservoir contenant du produit fluide à injecter, un piston étant disposé axialement déplaçable dans ledit réservoir et une aiguille pourvue d'une pointe d'injection étant fixée audit réservoir,
- une tige de piston déplaçable axialement par rapport audit réservoir et coopérant avec ledit piston lors de l'injection pour le déplacer axialement dans ledit réservoir,
- un manchon disposé autour de ladite seringue en étant axialement déplaçable par rapport audit réservoir entre une position de repos, dans laquelle ledit manchon ne recouvre pas ladite pointe d'injection de ladite aiguille, et une position projetée, dans laquelle ledit manchon recouvre ladite pointe d'injection de ladite aiguille, ledit manchon étant dans sa position de repos avant actionnement du dispositif d'injection,
ledit manchon étant déplacé vers sa position projetée après injection dudit produit fluide, ledit déplacement dudit manchon étant réalisé par ladite tige de piston.

Avantageusement, en position de repos ledit manchon est relié à ladite seringue, notamment par des ponts sécables.

Avantageusement, un second piston est disposé axialement déplaçable dans le réservoir, ledit produit fluide à injecter étant disposé entre ledit piston et ledit second piston.

Avantageusement, ledit second piston est percé par ladite aiguille en début d'actionnement.

Avantageusement, ledit piston est percé par ladite aiguille en fin d'injection.

Selon l'invention, ledit manchon comporte des pattes internes qui s'étendent à l'intérieur de la seringue et des pattes ou un manchon externe qui s'étend à l'extérieur de la seringue.

Avantageusement, ledit dispositif est dépourvu de ressort.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
La figure 1 est une vue schématique en section transversale d'un dispositif d'injection selon un mode de réalisation avantageux, en position avant injection,
La figure 2 est une vue similaire à celle de la figure 1, en cours d'injection,
La figure 3 est une vue similaire à celle de la figure 2, en fin d'injection, avant déclenchement du dispositif de sécurité, et
La figure 4 est une vue similaire à celle de la figure 3, après déclenchement du dispositif de sécurité.

Le dispositif d'injection manuelle représenté sur les figures comporte une seringue comprenant un réservoir 1 contenant le produit à injecter, une aiguille 2 fixée audit réservoir 1 à travers laquelle le produit est distribué et un piston 3 adapté à se déplacer dans ledit réservoir 1 pour réaliser cette injection.

L'aiguille 2 comporte une pointe d'injection 20 qui est piquée dans la zone à injecter lors de l'utilisation.

Une tige de piston 5 coopère avec ledit piston 3 lors de l'injection pour le déplacer dans le réservoir 1.

La seringue peut classiquement être pourvue d'une collerette radiale 9 sur laquelle l'utilisateur va prendre appui lorsqu'il actionne la tige de piston 5.

Avantageusement, un second piston 4 est prévu dans le réservoir 1, le produit fluide à distribuer étant disposé entre le piston 3 et le second piston 4. Ce second piston 4 a pour fonction d'isoler le produit fluide avant utilisation, et il est percé par l'aiguille 2 en début d'actionnement. Ce second piston 4 est également déplaçable axialement dans ledit réservoir 1 lors de l'actionnement.

Un manchon 10 est prévu autour de la seringue, en étant déplaçable axialement par rapport à la seringue entre une position de repos et une position projetée. Ce manchon 10 comporte des pattes internes 11 qui s'étendent à l'intérieur de la seringue et des pattes ou un manchon externe 12 qui s'étend à l'extérieur de la seringue. Cette mise en oeuvre, qui minimise la quantité de matière du manchon 10 qui doit être logée à l'intérieur de la seringue, permet de simplifier la fabrication et l'assemblage. De plus, ceci permet de minimiser les dimensions, notamment radiales, de la seringue. Ainsi, alors que les dispositifs dans lesquels la totalité du manchon est reçue à l'intérieur de la seringue requièrent des dimensions suffisantes pour ce faire, la présente invention permet de réduire ces dimensions, puisque seules les pattes internes 11 du manchon 10 sont disposées à l'intérieur de la seringue.

Avant injection, le manchon 10 est disposé autour du réservoir 1 en position de repos, de manière à exposer la pointe d'injection 20 de l'aiguille 2. Il est à noter qu'avant utilisation, cette pointe d'injection 20 peut être protégée par un capuchon approprié (non représenté), que l'utilisateur retire lorsqu'il souhaite utiliser le dispositif d'injection.

Après l'injection, ledit manchon 10 est déplacé axialement par rapport au réservoir 1 vers sa position projetée, dans laquelle il est disposé autour de la pointe d'injection 20 de l'aiguille 2, pour éviter tout risque de blessure avec ladite aiguille 2, formant ainsi un dispositif de sécurité post-injection.

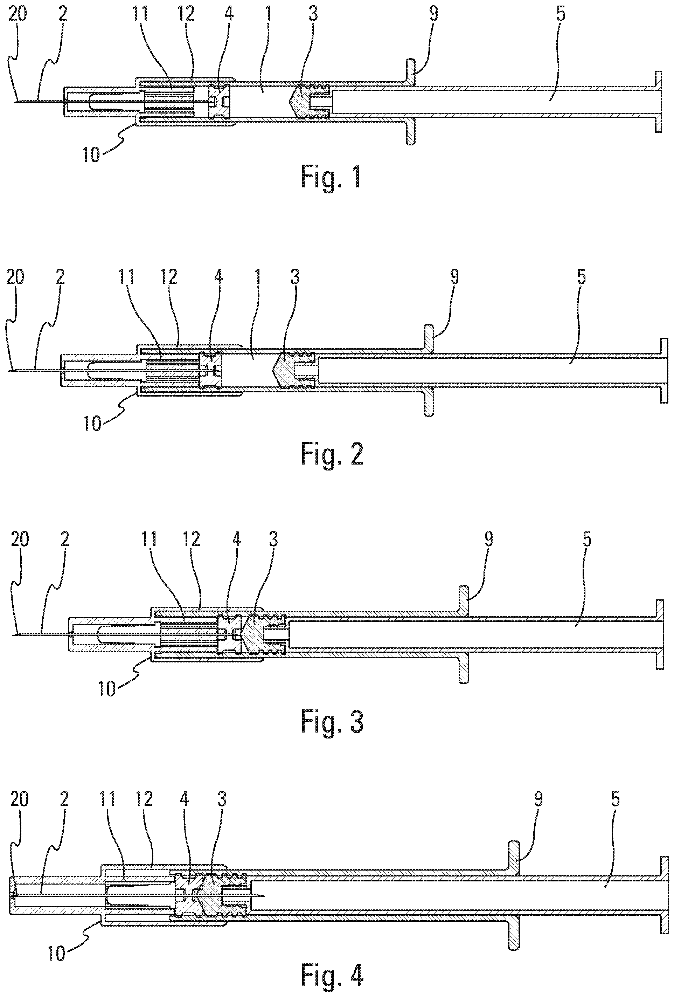

En référence aux figures 1 à 4, l'utilisateur qui souhaite utiliser le dispositif d'injection vient piquer le site d'injection avec la pointe d'injection 20 de l'aiguille 2, puis il exerce une pression axiale sur la tige de piston 5. Celle-ci se déplace axialement par rapport au réservoir 1, pour déplacer le piston 3 dans le réservoir 1. Ceci génère une pression dans le produit fluide qui, étant incompressible, transmet cette pression au second piston 4. Celui-ci est donc forcé à se déplacer axialement contre l'aiguille 2 qui vient ainsi le percer, comme visible sur la figure 2. A partir de ce moment, le déplacement axial de la tige de piston 5 déplace le piston 3 dans le réservoir 1, expulsant ainsi le produit fluide à travers l'aiguille 2 pour l'injecter dans le site d'injection.

En fin d'injection, le piston 3 arrive en butée contre le second piston 4, lui-même en butée contre les pattes internes 11 du manchon 10, comme visible sur la figure 3. Si l'utilisateur continue son appui axial sur la tige de piston 5, cette force d'appui va être transmise via le piston 3 et le second piston 4 audit manchon 10. Le manchon 10 est alors déplacé vers sa position projetée, dans laquelle il recouvre la pointe d'injection 20 de l'aiguille 2, pour éviter tout risque de blessure avec l'aiguille 2, comme visible sur la figure 4. Simultanément, l'aiguille 2, qui est fixe par rapport au réservoir 1, perce le piston 3 pour permettre ce déplacement axial du manchon 10.

Avantageusement, le manchon 10 peut être, en position de repos, relié à la seringue, par exemple par des ponts sécables réalisés par moulage. Ces ponts sécables sont cassés en fin d'injection, lorsque le manchon 10 est déplacé vers sa position projetée. Ainsi, on garantit que lors du déplacement axial de la tige de piston 5 par rapport au réservoir 1, on réalise d'abord l'injection du produit fluide à travers l'aiguille 2 et que le manchon 10 n'est déplacé qu'une fois l'injection terminée. D'autres moyens de liaison du manchon 10 à la seringue en position de repos sont possibles, comme par exemple le frottement ou un coincement mécanique.

Un avantage de la présente invention est l'absence de ressort et de patte(s) flexible(s), ce qui simplifie la fabrication et l'assemblage du dispositif, et rend son utilisation plus fiable.

Bien que la présente invention ait été décrite en référence à un mode de réalisation avantageux, il est entendu que l'homme du métier peut y apporter diverses modifications sans sortir du cadre de la présente invention tel que défini par les revendications annexées.

## Revendications

1. Dispositif d'injection de produit fluide comportant :
- une seringue comportant un réservoir (1) contenant du produit fluide à injecter, un piston (3) étant disposé axialement déplaçable dans ledit réservoir (1) et une aiguille (2) pourvue d'une pointe d'injection (20) étant fixée audit réservoir (1),
- une tige de piston (5) déplaçable axialement par rapport audit réservoir (1) et coopérant avec ledit piston (3) lors de l'injection pour le déplacer axialement dans ledit réservoir (1),
- un manchon (10) disposé autour de ladite seringue en étant axialement déplaçable par rapport audit réservoir (1) entre une position de repos, dans laquelle ledit manchon (10) ne recouvre pas ladite pointe d'injection (20) de ladite aiguille (2), et une position projetée, dans laquelle ledit manchon (10) recouvre ladite pointe d'injection (20) de ladite aiguille (2), ledit manchon (10) étant dans sa position de repos avant actionnement du dispositif d'injection,
**caractérisé en ce que** ledit manchon (10) est déplacé vers sa position projetée après injection dudit produit fluide, ledit déplacement dudit manchon (10) étant réalisé par ladite tige de piston (5), ledit manchon (10) comportant des pattes internes (11) qui s'étendent à l'intérieur de la seringue et des pattes ou un manchon externe (12) qui s'étend à l'extérieur de la seringue.

2. Dispositif selon la revendication 1, dans lequel en position de repos ledit manchon (10) est relié à ladite seringue, notamment par des ponts sécables.

3. Dispositif selon la revendication 1 ou 2, dans lequel un second piston (4) est disposé axialement déplaçable dans le réservoir (1), ledit produit fluide à injecter étant disposé entre ledit piston (3) et ledit second piston (4).

4. Dispositif selon la revendication 3, dans lequel ledit second piston (4) est percé par ladite aiguille (2) en début d'actionnement.

5. Dispositif selon l'une quelconque des revendications précédentes, dans lequel ledit piston (3) est percé par ladite aiguille (2) en fin d'injection.

6. Dispositif selon l'une quelconque des revendications précédentes, dans lequel ledit dispositif est dépourvu de ressort.

## Patentansprüche

1. Fluidproduktinjektionsvorrichtung, aufweisend:
- eine Spritze, die einen Vorratsbehälter (1) aufweist, der zu injizierendes Fluidprodukt enthält, einen Kolben (3), der axial verschiebbar in dem Vorratsbehälter (1) angeordnet ist und eine Nadel (2), die mit einer Injektionsspitze (20) versehen ist, die an dem Vorratsbehälter (1) befestigt ist,
- eine Kolbenstange (5), die in Bezug auf den Vorratsbehälter (1) axial verschiebbar ist und bei der Injektion mit dem Kolben (3) zusammenwirkt, um ihn in dem Vorratsbehälter (1) axial zu verschieben,
- eine Hülse (10), die um die Spritze herum angeordnet ist, wobei sie in Bezug auf den Vorratsbehälter (1) zwischen einer Ruheposition, in der die Hülse (10) die Injektionsspitze (20) der Nadel (2) nicht bedeckt, und einer vorgeschobenen Position, in der die Hülse (10) die Injektionsspitze (20) der Nadel (2) bedeckt, axial verschiebbar ist, wobei sich die Hülse (10) vor der Betätigung der Injektionsvorrichtung in ihrer Ruheposition befindet,
**dadurch gekennzeichnet, dass** die Hülse (10) nach Injektion des Fluidprodukts in ihre vorgeschobene Position verschoben wird, wobei die Verschiebung der Hülse (10) durch die Kolbenstange (5) bewirkt wird, wobei die Hülse (10) innere Laschen (11), die sich im Inneren der Spritze erstrecken, und äußere Laschen oder eine äußere Hülse (12) aufweist, die sich außerhalb der Spritze erstreckt.

2. Vorrichtung nach Anspruch 1, wobei die Hülse (10) in Ruheposition mit der Spritze verbunden ist, insbesondere durch durchtrennbare Brücken.

3. Vorrichtung nach Anspruch 1 oder 2, wobei ein zweiter Kolben (4) axial verschiebbar in dem Vorratsbehälter (1) angeordnet ist, wobei das zu injizierende Fluidprodukt zwischen dem Kolben (3) und dem zweiten Kolben (4) angeordnet ist.

4. Vorrichtung nach Anspruch 3, wobei der zweite Kolben (4) bei Beginn der Betätigung von der Nadel (2) durchstochen wird.

5. Vorrichtung nach einem der vorhergehenden Ansprüche, wobei der Kolben (3) am Ende der Injektion von der Nadel (2) durchstochen wird.

6. Vorrichtung nach einem der vorhergehenden Ansprüche, wobei die Vorrichtung keine Feder aufweist.

## Claims

1. A fluid product injection device comprising:
- a syringe comprising a reservoir (1) containing fluid product to be injected, a piston (3) being arranged so as to be axially movable in said reservoir (1) and a needle (2) provided with an injection tip (20) being attached to said reservoir (1),
- a piston rod (5) axially movable relative to said reservoir (1) and cooperating with said piston (3) during injection in order to move it axially in said reservoir (1),
- a sleeve (10) arranged around said syringe while being axially movable relative to said reservoir (1) between a rest position, in which said sleeve (10) does not cover said injection tip (20) of said needle (2), and a projecting position, in which said sleeve (10) covers said injection tip (20) of said needle (2), said sleeve (10) being in its rest position before actuation of the injection device,
**characterized in that** said sleeve (10) is moved towards its projecting position after injection of said fluid product, said movement of said sleeve (10) being performed by said piston rod (5), said sleeve (10) including internal tabs (11) that extend inside the syringe and tabs or an outer sleeve (12) that extends outside the syringe.

2. The device according to claim 1, wherein, in its rest position, said sleeve (10) is connected to said syringe, in particular by breakable bridges.

3. The device according to claim 1 or claim 2, wherein a second piston (4) is arranged to move axially in the reservoir (1), said fluid product to be injected being arranged between said piston (3) and said second piston (4).

4. The device according to claim 3, wherein said second piston (4) is perforated by said needle (2) at the start of actuation.

5. The device according to any preceding claim, wherein said piston (3) is perforated by said needle (2) at the end of injection.

6. The device according to any one of preceding claims, wherein said device does not have a spring.
